# FASCICULE DE BREVET EUROPEEN

(11) **EP 3 275 129 B1**
(45) Date de publication et mention de la délivrance du brevet: **18.07.2018**
(21) Numéro de dépôt: 16716493.8
(22) Date de dépôt: 24.03.2016
(51) Int. Cl.: H04L 12/28, H04L 29/08

(54) **SYSTEME DE COMMUNICATION UNIVERSELLE POUR APPAREILS DE MESURE, PROCEDE DE COMMUNICATION S'Y RAPPORTANT**
UNIVERSELLES KOMMUNIKATIONSSYSTEM FÜR MESSVORRICHTUNGEN, VERFAHREN ZUR KOMMUNIKATION DAFÜR
UNIVERSAL COMMUNICATION SYSTEM FOR MEASUREMENT APPARATUSES, METHOD OF COMMUNICATION RELATING THERETO

(30) Priorité: 25.03.2015 FR 1552497
(43) Date de publication de la demande: 31.01.2018
(73) Titulaire: Ecomesure, 91400 Saclay (FR)
(72) Inventeur: PELLETIER, Damien, 91400 Orsay (FR); NEVEU, Cédric, 92120 Montrouge (FR); FURLAN, Fabio, 91240 Saint Michel Sur Orge (FR)
(74) Mandataire: Pontet Allano & Associes
(86) Numéro de dépôt international: PCT/EP2016/056602
(87) Numéro de publication internationale: WO 2016/151096

(56) Documents cités:
- US-A1- 2003 187 920
- US-A1- 2011 202 189
- US-A1- 2013 046 412
- US-A1- 2013 282 196

## Description

### Domaine technique

La présente invention concerne système de communication universelle pour des instruments de mesure.

La présente invention se situe dans le domaine de la mise en réseau d'instruments de mesure.

### Etat de la technique antérieure

Dans le domaine particulier de la métrologie des aérosols et de la qualité de l'air, les appareils de mesure de la qualité de l'air et de la présence d'aérosols dans l'air sont très répandus sur les territoires urbains. Il existe ainsi un très grand nombre d'appareils de mesure permettant de mesurer des nombreux paramètres relatifs à la qualité de l'air et aux aérosols présents, afin de déterminer notamment des niveaux de pollution et des seuils d'alerte.

De très nombreux acteurs proposent des appareils de mesure intégrant des capteurs dont les technologies sont variées et les protocoles de communication parfois spécifiques. Ainsi, il est très difficile de regrouper l'ensemble des données mesurées par ces différents appareils de mesure au sein d'un même réseau informatique.

On connait des solutions d'interconnexion de ces appareils de mesure à l'aide d'ordinateurs équipés de cartes de communication, de moyens de stockage et de logiciels complexes intégrant l'ensemble des protocoles de communication avec lesdits appareils de mesure. Ces solutions sont dites « client lourd » car l'ensemble des logiciels et des protocoles de communication sont intégrés sur lesdits ordinateurs qui sont connectés aux appareils de mesure. Il est ainsi nécessaire de compléter chaque instrument de mesure par un ordinateur, et les logiciels et protocoles de communication doivent être adaptés à chaque instrument de mesure. La publication de brevet US 2013/0046412 décrit ainsi un réseau de gestion d'énergie comprenant une passerelle et des appareils de mesure. Les inconvénients associés aux solutions actuelles sont le coût élevé de l'infrastructure nécessaire (chaque instrument de mesure étant associé à un ordinateur), et un niveau de fiabilité limité par l'architecture matérielle. De plus, l'ajout d'un nouveau capteur non géré nécessite une mise à jour locale sur chaque poste connecté aux appareils de mesure. Enfin, ces solutions nécessitent un niveau de maintenance élevé, notamment du fait de l'interface informatique, de l'architecture logicielle et de ses mises à jour.
Dans des domaines plus large de l'instrumentation scientifique (mesures de la qualité de l'eau, de niveaux de bruits, de trafic, d'odeurs, mesures sismiques, météorologiques...) et de la mise en réseau d'instruments de mesure délocalisés et n'intégrant pas des moyens de télécommunication suffisant, les problématiques évoquées ci-dessus restent valables.
La présente invention a pour objet de répondre au moins en grande partie aux problèmes précédents et de conduire en outre à d'autres avantages.
Un autre but de l'invention est de résoudre au moins un de ces problèmes par un nouveau système de communication entre appareils de mesure, notamment dans le domaine de la métrologie des aérosols et de la qualité de l'air.
Un autre but de la présente invention est de réduire les coûts et la maintenance associés à l'architecture réseau de tels appareils de mesure. Un autre but de la présente invention est d'améliorer la fiabilité d'une telle architecture réseau.
Un autre but de l'invention est d'améliorer l'accessibilité des données mesurées par les appareils de mesure, et notamment ceux du domaine de la métrologie des aérosols et de la qualité de l'air.

### Exposé de l'invention

Suivant un premier aspect de l'invention, on atteint au moins l'un des objectifs précités avec un système de communication universelle pour appareils de mesure, notamment ceux dédiés à la métrologie de la qualité de l'air et des aérosols, ledit système étant défini dans la revendication 1. La présente invention vise particulièrement - mais non exclusivement - la mise en réseau et la communication entre instruments de mesures de la qualité de l'air et des aérosols. Cependant, d'autres domaines d'utilisation sont adressés par la présente invention, notamment celui des mesures de la qualité de l'eau, des mesures de bruit, de trafic, d'odeurs, les mesures sismiques ou radioactives, les mesures météorologiques (température, pression, hygrométrie...)
Il est ainsi possible de mettre en réseau une pluralité d'instruments de mesure à l'aide d'un système à faible coût : l'architecture logicielle et matérielle est concentrée à un noeud de communication qui est agencé et configuré pour communiquer - en aval - avec n'importe quel instrument de mesure grâce à de nombreux moyens de communications différents, lui permettant de s'adapter à de très nombreux types d'instruments de mesure et de protocoles de communication ; et - en amont - avec n'importe quel client disposant d'une connexion internet. La fiabilité de la nouvelle solution proposée par la présente invention est aussi plus fiable que les solutions connues jusqu'alors.

La maintenance est ainsi facilitée puisqu'il suffit d'intervenir en un seul lieu pour réparer d'éventuelles défaillances. Grâce à une connexion distante, il est aussi possible de mettre à jour l'infrastructure logicielle afin de par exemple pouvoir communiquer avec de nouveaux instruments de mesure et/ou selon de nouveaux protocoles de communication.

Enfin, grâce à la connexion internet et au serveur distant, il est désormais possible d'accéder à un très grand nombre d'informations relatives aux instruments de mesures et depuis n'importe quel endroit, sans avoir à se déplacer pour télécharger les données enregistrées par un instrument de mesure particulier (et/ou un peu ancien, sans moyens de communications modernes...), sans avoir besoin d'utiliser un logiciel particulier, et de manière centralisée, un système selon l'invention permettant d'adresser une pluralité d'instruments de mesure. L'accessibilité des données mesurées - entre autres - est ainsi améliorée.

Le système selon l'invention comprend ainsi deux aspects fondamentaux : une communication avale vers les instruments de mesure et une communication amont via internet pour mettre à disposition un certain nombre de données relatives aux instruments de mesure.

La communication avale entre la passerelle et les instruments de mesure permet de transporter les données relatives à chaque instrument de mesure sans en interpréter le contenu. Il s'agit d'une communication bidirectionnelle et lesdites données peuvent être de tout type. A titre d'exemple non limitatif, il peut s'agir de données relatives aux mesures physiques réalisées par lesdits instruments de mesure, de paramètres de configuration desdits instruments de mesure transmis alors par la passerelle vers l'instrument de mesure, de mises à jour logicielle desdits instruments de mesure...

La passerelle comprend une architecture matérielle et logicielle lui permettant de se comporter comme un serveur web local. Elle peut comprendre par ailleurs les toutes dernières technologies de connexion et de sécurisation de flux des données (Firewall, SSL, SSH, ...).

De manière préférentielle, le système de communication selon l'invention utilise des technologies industrielles robustes et des moyens de stockage du type mémoire flash plutôt que disque dur rotatif. L'unité de traitement peut être de tout type connu, tel que par exemple microprocesseur, microcontrôleur... d'une manière générale, la passerelle comprend une réserve de puissance logicielle et matérielle qui permet d'évoluer et de l'adapter aux besoins spécifiques des instruments de mesure et d'intégrer d'éventuels modules complémentaires futurs.

La communication amont vers internet complète la communication avale et permet d'établir une interconnexion physique et logicielle entre n'importe quel type d'instrument de mesure via la passerelle et le serveur distant suivant un très grand nombre de moyens de communication sans ajout de logiciel et/ou de protocole de communication.

Le serveur distant est configuré pour stocker toutes les données relatives aux instruments de mesure inclus dans le réseau formé par le système selon l'invention.

De manière préférentielle, il peut comprendre toutes les technologies connues pour sécuriser le stockage et les accès audites données mémorisées. A titre d'exemples non limitatifs, l'accès au serveur peut être sécurisé par un accès avec identifiant et mot de passe, les données enregistrées peuvent être cryptées.

Le serveur distant comprend aussi une plateforme distante accessible en mode Software As A Service (SAAS, pour logiciel en tant que service).

La plateforme est du type client léger, c'est-à-dire qu'elle est accessible depuis un simple navigateur internet sur n'importe quel support informatique (tablette, ordinateur, smartphone...) et sans installation de logiciel particulier.

De manière préférentielle, la plateforme distante est architecturée sur la base des technologies connues pour des plateformes web à fort trafic. Elle est aussi configurée pour être répliquée à tout moment pour des sauvegardes, des transferts, des montées en puissance de l'unité de traitement, notamment par des ajouts d'unités de stockage.

Avantageusement, la plateforme distante est configurée pour cloisonner les données stockées afin de renforcer la sécurité.

La plateforme distante constitue l'intelligence du système selon l'invention et, en particulier, elle comprend les protocoles de communications avec les appareils de mesure. Si nécessaire, de nouveaux protocoles de communication peuvent être ajoutés à la plateforme via internet.

Il est ainsi possible depuis la plateforme distante d'avoir accès à un très grand nombre de données relatives aux instruments, mais aussi de les paramétrer spécifiquement. A titre d'exemples non limitatifs, la plateforme distante permet notamment d'afficher en temps réel l'état des instruments de mesure et des mesures réalisées, d'afficher et de télécharger les données relatives à chaque instrument et/ou l'historique correspondant, de définir des alarmes de fonctionnement et/ou de dépassement de certain seuils paramétrables, d'éditer et télécharger des rapports de synthèse relatifs à chaque instrument, de prendre le contrôle à distance, de réaliser de la télémaintenance, de piloter des entrées/sorties et/ou de réaliser des transferts ftp automatisés.

Le serveur distant est configuré par ailleurs pour permettre à l'utilisateur d'ajouter des nouvelles fonctionnalités ou de nouveaux appareils de mesure directement depuis le serveur distant, ce qui permet une mise à jour centralisée pour tous les clients de la plateforme distante et donc des frais d'utilisation et de maintenance réduits.

Avantageusement, dans un système de communication universelle conforme au premier aspect de l'invention, les premiers moyens de connexion peuvent être du type Ethernet et/ou RS232 et/ou USB et/ou sans fils. L'unité de traitement comprise dans la passerelle est configurée pour stocker les protocoles de communication avec les instruments de mesure et relatifs auxdits premiers moyens de communication.

De manière préférentielle, dans un système de communication universelle conforme au premier aspect de l'invention, les seconds moyens de connexion peuvent être du type filaire ou sans fils. A titre d'exemple non limitatifs, les modes de connexion avec le réseau distant - et donc vers internet - peuvent être du type Ethernet, Wifi, ou selon n'importe quelle norme de télécommunication : GPRS, 2G, 3G, 4G...

De manière avantageuse, dans un système de communication universelle conforme à l'un quelconque des modes de réalisation du premier aspect de l'invention, les données envoyées vers le serveur distant peuvent être encapsulées et/ou cryptées.

D'une manière générale, un protocole de communication particulier est défini entre la passerelle et le serveur distant, permettant l'échange et l'encapsulation des données sans en interpréter le contenu. Le protocole de communication particulier fournit ainsi un conteneur de transport des données échangées entre les passerelles et le serveur distant. Par ailleurs, il assure aussi la désencapsulation des données et protocoles transférés depuis le serveur distant vers les instruments de mesure. L'encapsulation et la désencapsulation sont réalisées à la fois par chaque passerelle et par la plateforme distante.

Le serveur distant dernier comprend les commandes d'adressage des instruments de mesure locaux. Les commandes d'adressage traversent les couches internet pour arriver à une passerelle qui transfère ensuite lesdites commandes aux instruments de mesure concernés.

Ainsi, le protocole d'encapsulation comprend à la fois l'adresse internet de la passerelle au travers de laquelle les informations doivent être acheminées, et d'autre part la liaison entre l'instrument de mesure et ladite passerelle par laquelle ils sont reliés.

Dans le sens descendant (serveur distant vers instrument de mesure), la commande encapsulée est reçue par la passerelle qui désencapsule ladite commande sans l'interpréter et la transfère directement vers l'instrument de mesure par la liaison désignée.

Dans le sens ascendant (instrument de mesure vers serveur distant), l'instrument répond à la commande vers la passerelle, qui encapsule la réponse pour la renvoyer à la plateforme distante qui est, préférentiellement, seule apte à interpréter les données contenues dans la réponse.

Avantageusement, le protocole de communication entre le serveur distant et les passerelles est sécurisé et crypté selon les technologies connues du domaine civil.

De manière préférentielle, plusieurs passerelles peuvent être interconnectées au serveur distant de façon cryptée et sécurisée et selon une technique connue de tunneling.

Préférentiellement, le protocole de communication particulier de la présente invention comprend des fonctions supplémentaires, configurées pour surveiller et contrôler chaque passerelle du réseau ainsi formé.

De manière avantageuse, les données transitant entre les passerelles et le serveur distant ne sont pas stockées localement mais sur le serveur distant afin de bénéficier des fonctions de sécurité informatique et de permettre un accès permanent aux données sans devoir réinterroger les instruments de mesure locaux.

Eventuellement, chaque passerelle comprend des moyens de stockage configurés pour enregistrer temporairement des données des instruments de mesure. Ce stockage local et temporaire permet ainsi de faire face à d'éventuelles coupures de connexion internet et d'enregistrer les données des instruments de mesure pendant cette interruption de connexion. Lorsque la connexion est rétablie, les données sont transférées vers le serveur distant.

Selon un mode de réalisation préférée conforme à l'un quelconque des modes de réalisation du premier aspect de l'invention, la plateforme distante peut être configurée en outre pour directement contrôler à distance au moins un instrument de mesure, préférentiellement par un client léger du type fenêtre virtuelle émulant l'interface de contrôle dudit au moins un instrument de mesure. Pour ce faire, le serveur distant et/ou la plateforme distante stockent tous les protocoles de communications propres à chaque instrument de mesure afin de pouvoir communiquer avec eux et échanger des données.

En effet, dans le cas où l'instrument de mesure comprend des commandes logicielles permettant sa prise de contrôle, la plateforme distante est configurée pour piloter ledit instrument de mesure à distance et pour remonter les données au serveur distant et via une interface utilisateur standardisée. Dans le cas où l'instrument de mesure ne comprend pas de commandes permettant sa prise de contrôle, un système de prise de contrôle de l'interface graphique de l'instrument de mesure est mis en place afin de disposer de la fenêtre de contrôle de l'appareil sur la plateforme internet sans ajout de logiciel sur le poste client. Cette interface graphique particulière est mise au point au moment de l'intégration d'un tel instrument de mesure dans le réseau formé par la présente invention.

Eventuellement, dans un système de communication universelle conforme à l'un quelconque des modes de réalisation du premier aspect de l'invention, au moins une passerelle peut comprendre un moyen de géolocalisation manuel ou automatique. Il est ainsi possible de réaliser des traitements statistiques sur des sous-ensembles d'appareils de mesure et d'établir des corrélations particulières entre les données mesurées d'une part et la localisation des instruments de mesure d'autre part. Ces corrélations sont très importantes dans le domaine particulier de la métrologie de la qualité de l'air. Pour des instruments de mesure ne disposant pas de moyens internes de géolocalisation, la géolocalisation de la passerelle peut être un moyen suffisant pour réaliser de telles corrélations.

Si l'instrument de mesure comprend des moyens internes de géolocalisation, alors les informations de géolocalisation sont automatiquement transférées via la passerelle vers le serveur distant et/ou la plateforme distante. En revanche, si l'instrument de mesure ne comprend pas de moyens internes de géolocalisation, alors l'information de géolocalisation est enregistrée manuellement dans le serveur distant et/ou la plateforme distante, par exemple lors de l'ajout dudit instrument de mesure audit système selon l'invention.

Selon un autre mode de réalisation de l'invention, conforme à n'importe lequel des modes de réalisation du premier aspect de l'invention comprenant une pluralité de passerelles, au moins une première partie de la pluralité de passerelles peut être agencée pour communiquer avec la plateforme distante, et au moins une deuxième partie de la pluralité de passerelles peut être connectée à au moins une passerelle de ladite première partie par les premiers moyens de connexion.

A titre d'exemple non limitatif, chaque passerelle peut être configurée pour se connecter à une autre passerelle au sein d'un réseau local, par des moyens de communication filaire ou non filaire (radio). Cette configuration avantageuse permet ainsi de définir un sous-réseau de communication vers des instruments de mesure, par exemple dans des zones d'accès difficiles et/ou en dehors de tout réseau de télécommunication. Ce sous réseau peut ensuite être avantageusement complété par une passerelle qui est connectée à la fois à ce sous-réseau (par les moyens de communication locaux) et à internet (par les moyens de communication distants). Il est ainsi possible de relayer les données des appareils de mesure dudit sous-réseau vers internet, et plus particulièrement vers le serveur distant afin de rendre accessible lesdites données.

Selon un deuxième aspect de l'invention, il est proposé un procédé de transfert de données d'instruments de mesure, ledit procédé mettant en oeuvre le système selon des modes de réalisation du premier aspect de l'invention, ledit procédé comprenant au moins une itération des étapes suivantes :
- transfert des données d'un instrument de mesure vers la passerelle, par l'intermédiaire des premiers moyens de connexion,
- encapsulation desdites données de mesures par ladite passerelle,
- transfert des données encapsulées vers le serveur distant grâce aux seconds moyens de connexion.

Eventuellement, l'encapsulation peut comprendre une étape de cryptage des données. Par ailleurs le transfert des données encapsulées peut se faire, comme décrit précédemment, suivant des protocoles de communication sécurisés connus.

De manière avantageuse, le procédé de transfert de données conforme au deuxième aspect de l'invention peut comprendre une étape de paramétrage d'au moins un instrument de mesure par accès distant :
- affichage d'une interface graphique de type client web configurée pour définir des variables de paramétrage dudit instrument,
- transfert desdites variables de paramétrage vers la passerelle par les seconds moyens de connexion et via un protocole de transfert sécurisé,
- transfert desdites variables de paramétrage vers ledit instrument de mesure par les premiers moyens de connexion.

Eventuellement, le paramétrage de l'au moins un instrument de mesure peut se faire par un contrôle à distance et/ou par le biais d'une fenêtre virtuelle, comme décrit précédemment.

Préférentiellement, le procédé conforme à l'une quelconque des versions du deuxième aspect de l'invention peut comprendre une étape de téléchargement sur la plateforme distante d'un protocole de mesure agencé pour communiquer avec au moins un instrument de mesure.

De manière avantageuse, les protocoles sont stockés sur la plateforme distante exclusivement. Cependant, dans le cas d'une perte de réseau internet, une commande spécifique à chaque instrument de mesure, définie par la plateforme distante et stockée préalablement sur la passerelle permet, lors de son exécution, de stocker d'une part des données provenant desdits instruments de mesure durant la durée de la coupure et d'autre part de les restituer vers la plateforme distante une fois que la liaison internet est rétablie.
En particulier, le système selon l'invention peut comprendre :
- un serveur distant comprenant :
- des moyens de stockage,
- une plateforme distante du type client léger et configurée pour interagir avec au moins une partie d'une pluralité d'instruments de mesure,
- au moins une passerelle disposée entre le serveur distant et la pluralité d'instruments de mesure, ladite au moins une passerelle comprenant :
- des premiers moyens de connexion bidirectionnelle avec la pluralité d'instruments de mesure,
- des seconds moyens de connexion bidirectionnelle avec le serveur distant,
- une unité de traitement configurée pour communiquer avec lesdits instruments de mesure selon une pluralité de protocoles de communication, ladite unité de traitement étant agencée pour stocker et exécuter au moins une application logicielle.
Selon l'invention, les protocoles de communication avec les appareils de mesure sont exclusivement stockés sur la plateforme distante. Par ailleurs, l'unité de traitement est configurée pour stocker dans la passerelle une commande spécifique à chaque instrument de mesure et définie par la plateforme distante, cette commande permettant en cas de coupure internet de stocker d'une part des données provenant desdits instruments de mesure durant la durée de la coupure et d'autre part de les restituer vers la plateforme distante une fois que la liaison internet est rétablie.

Selon un mode de réalisation avantageux de l'invention, chaque protocole de communication de tout ou partie des appareils de mesure comporte d'une part des couches de présentation et d'application, telles que par exemple tout ou partie des couches hautes dans le modèle OSI, et d'autre part des couches physiques et de liaisons, telles que par exemple des couches matérielles dans le modèle OSI. Les couches de présentation et d'application peuvent comprendre la syntaxe et la sémantique des instruments de mesure comme par exemple dans le protocole Modbus, le protocole AK ou des protocoles propriétaires.

En complément de ce qui précède, selon l'invention, la plateforme peut être configurée pour communiquer avec les instruments de mesure selon une pluralité de protocoles de communication au niveau des couches de présentation et d'application. Par ailleurs, l'unité de traitement de la passerelle peut communiquer avec les appareils de mesure selon une pluralité de protocoles de communication au niveau des couches physiques et de liaisons de données.

Autrement, chaque instrument de mesure peut être doté d'une application logicielle dédiée pour exploiter des données brutes provenant de capteurs internes de cet instrument. Selon l'invention, cette application logicielle dédiée peut être exclusivement stockée dans le serveur distant et non sur la passerelle qui ne joue alors qu'un rôle de transfert de données brutes provenant de capteurs internes de l'instrument, sans capacité de les exploiter. Pour chaque appareil, cette application peut être une interface graphique utilisateur dédiée.

Les commandes des appareils proviennent directement du serveur distant sans interprétation par la passerelle. Dans ce cas, l'invention est particulièrement avantageuse car pour chaque appareil de mesure, le protocole (par exemple idéalement tout le protocole, pas uniquement les couches hautes) de communication avec cet appareil est installé dans le serveur distant et non dans la passerelle. Cette dernière comporte uniquement les couches matérielles permettant de rentrer en contact avec l'appareil. Les données sont récupérées et interprétées au sein du serveur distant et non dans la passerelle. L'intelligence se trouve dans le serveur distant. La passerelle ne fait que récupérer des trames provenant de l'appareil et les transférer vers le serveur distant sans lecture des données. Avec une telle passerelle transparente, le serveur distant peut accéder aisément à un ensemble d'appareils de mesure sans paramétrage de la passerelle, seul le serveur distant intègre l'ensemble des protocoles. La liaison entre le serveur distant et la passerelle est un tuyau permettant de faire passer les commandes provenant du serveur vers les appareils.
En d'autres termes, le système selon l'invention peut comprendre :
- un serveur distant comprenant :
- des moyens de stockage,
- une plateforme distante du type client léger et configurée pour interagir avec au moins une partie d'une pluralité d'instruments de mesure,
- au moins une passerelle disposée entre le serveur distant et la pluralité d'instruments de mesure, ladite au moins une passerelle comprenant :
   - des premiers moyens de connexion bidirectionnelle avec la pluralité d'instruments de mesure,
   - des seconds moyens de connexion bidirectionnelle avec le serveur distant,
   - une unité de traitement configurée pour communiquer avec lesdits instruments de mesure selon une pluralité de protocoles de communication, ladite unité de traitement étant agencée pour stocker et exécuter au moins une application logicielle.
Selon l'invention, les protocoles de communication avec les appareils de mesure sont exclusivement stockés sur la plateforme distante, le serveur étant configuré pour générer des commandes au niveau des couches hautes des protocoles de communication avec les appareils, et la passerelle étant configurée pour communiquer avec les appareils au niveau uniquement des couches matérielles des protocoles de communication avec les appareils. Par ailleurs, l'unité de traitement est configurée pour stocker temporairement dans la passerelle une commande spécifique à chaque instrument de mesure et définie par la plateforme distante, cette commande permettant en cas de coupure internet de stocker d'une part des données provenant desdits instruments de mesure durant la durée de la coupure et d'autre part de les restituer vers la plateforme distante une fois que la liaison internet est rétablie.
En fait la passerelle est configurée pour enregistrer de façon temporaire des commandes provenant du serveur distant et de s'en servir en cas de coupure de connexion.
Par temporaire, on entend par exemple une durée pendant laquelle le serveur distant est en communication avec un appareil, cette durée peut être complétée par la durée liée à la coupure de connexion.

### Description des figures et des modes de réalisation

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :
- la figure 1 illustre un système de communication universelle pour instruments de mesure,
- la figure 2 illustre une architecture réseau particulière du système de communication universelle selon l'invention,
- la figure 3 illustre un vue de la plateforme distante représentant les mesures réalisées par un instrument de mesure, et
- la figure 4 illustre une fenêtre virtuelle pour le contrôle à distance d'un instrument de mesure.

Les modes de réalisation qui seront décrits dans la suite ne sont nullement limitatifs ; on pourra notamment imaginer des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier toutes les variantes et tous les modes de réalisation décrits sont combinables entre eux si rien ne s'oppose à cette combinaison sur le plan technique.

Sur les figures, les éléments communs à plusieurs figures conservent la même référence.

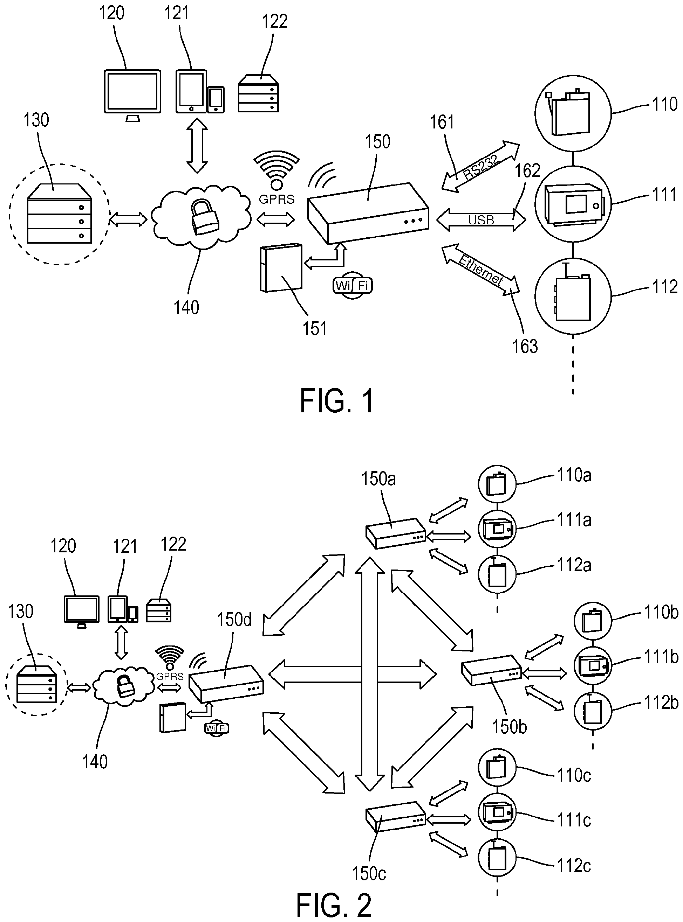

La FIGURE 1 décrit un système de communication universelle pour instruments de mesure, notamment des instruments de mesure de la qualité de l'air, et dans lequel une pluralité d'instruments de mesure 110-112 sont reliés à une passerelle 150 via des connexions locales du type RS232, USB, Ethernet... La passerelle 150 comprend ainsi des premiers moyens de communication 161-163 bidirectionnels et compatibles avec la pluralité d'instruments de mesure.

La passerelle comprend des seconds moyens de connexion bidirectionnels 151, 152 agencés et configurés pour permettre à ladite passerelle 150 de se connecter à internet.

La connexion à internet 140 se fait avantageusement par des moyens sécurisés.

Un serveur distant 130 comprenant des moyens de stockage et une plateforme distante non représentés met ainsi à disposition de clients 120-122 les données de mesure envoyées par lesdits instruments de mesure 110-112 via la passerelle 150. Comme décrit précédemment, la plateforme distante est configurée pour permettre la prise de contrôle à distance et/ou le paramétrage des instruments de mesure 110-112.

Les clients accèdent eux aussi de manière sécurisée 140 au serveur distant 130, et grâce à un simple navigateur web, sans application logicielle particulière. Ainsi l'accès au serveur distant 130 et à la plateforme est possible depuis un ordinateur 120, un smartphone ou une tablette 121 ou un serveur ftp 122, plateformes internet API...

La FIGURE 2 illustre une architecture réseau particulière du système de communication universelle selon l'invention, dans laquelle un sous-réseau local regroupant plusieurs groupes d'instruments est interfacé par une passerelle disposant - elle - d'un accès à internet.

Chaque passerelle 150a, 150b, 150c du sous réseau interface ainsi une pluralité d'instruments de mesure, respectivement 110a, 111a, 112a, 110b, 111b, 112b et 110c, 111c, 112c.

Chaque passerelle 150a, 150b, 150c communique avec toutes les autres 150a, 150b, 150c par le biais d'un réseau local et via les premiers moyens de communication.

De plus, chaque passerelle 150a, 150b, 150c communique aussi avec la passerelle 150d par les premiers moyens de communication.

La passerelle 150d est agencée et configurée pour d'une part communiquer avec les passerelles 150a, 150b, 150c du sous-réseau par des moyens de communication locaux, et d'autre part pour communiquer via internet et des seconds moyens de communication distants.

La passerelle 150d permet ainsi de communiquer avec le serveur distant 130 via un accès sécurisé 140, et permet ainsi à toutes les passerelles du sous-réseau de communiquer, via elle, avec internet et d'avoir un accès au serveur distant. Ainsi, l'ensemble des instruments de mesure du sous réseau qui, au départ, n'étaient pas connectés à internet, peuvent grâce à cette architecture réseau être connectés au serveur distant, à la plateforme distante, et bénéficier de l'ensemble des fonctionnalités de la présente invention.

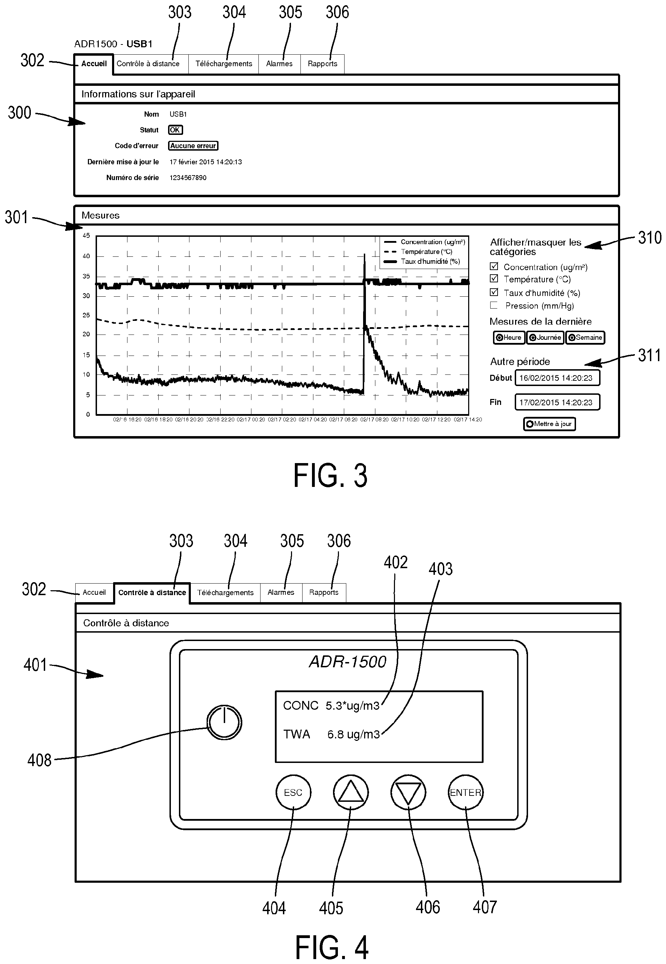

La FIGURE 3 illustre un vue de la plateforme distante représentant les mesures réalisées par un instrument de mesure.

Plusieurs onglets 302-306 permettent de sélectionner différentes fonctionnalités de visualisation sur le client web : le contrôle à distance 303 de l'instrument de mesure, le téléchargement 304 des mesures réalisées, la définition d'alarmes 305 et la génération de rapports 306.

L'onglet accueil 302 est celui activé sur la FIGURE 3. Il met en avant un premier cadre 300 relatif aux informations de l'instrument de mesure dont les données sont affichées à l'écran (le nom, son statut, un code d'erreur, un numéro de série et la date de la mise à jour des données).

Un deuxième cadre 301 affiche un graphique représentant les données de mesure pour une période donnée.

Deux zones de contrôle 310 et 311 permettent respectivement de sélectionner les courbes à afficher sur le graphique et de sélectionner la période de temps à afficher.

La FIGURE 4 illustre une fenêtre virtuelle pour le contrôle à distance d'un instrument de mesure disposant d'une interface logicielle. Cette interface est représentée dans l'onglet « Contrôle à Distance » 303, et plus particulièrement à l'intérieur d'un cadre 401.

Une interface utilisateur reproduisant l'interface graphique du logiciel de l'instrument de mesure dont le contrôle est pris via la plateforme distante est affichée par ladite plateforme distante. L'interface graphique représente la face avant de l'instrument de mesure, avec une série de boutons 404-408 permettant respectivement de sortir d'un menu, incrémenter une valeur numérique, décrémenter une valeur numérique, valider une valeur et éteindre l'instrument de mesure.

Sur l'écran simulé par l'interface graphique, deux paramètres 402 et 403 sont configurables. La modification des valeurs de chaque paramètre, une fois validée, sera transmise à l'instrument de mesure via le réseau et via le protocole de communication du système de communication universelle d'une part, via le protocole de communication de l'instrument de mesure d'autre part, et via la passerelle.

Bien sûr, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Système de communication universelle (100) pour appareils de mesure, ledit système comprenant :
- un serveur distant (130) comprenant :
- des moyens de stockage,
- une plateforme distante du type client léger et configurée pour interagir avec au moins une partie d'une pluralité d'instruments de mesure (110-112),
- au moins une passerelle (150) disposée entre le serveur distant (130) et la pluralité d'instruments de mesure (110-112), ladite au moins une passerelle (150) comprenant :
- des premiers moyens de connexion (161-163) bidirectionnelle avec la pluralité d'instruments de mesure (110-112),
- des seconds moyens de connexion (151-152) bidirectionnelle avec le serveur distant (130),
- une unité de traitement configurée pour communiquer avec lesdits instruments de mesure (110-112) selon une pluralité de protocoles de communication, ladite unité de traitement étant agencée pour stocker et exécuter au moins une application logicielle,
**caractérisé en ce que** les protocoles de communication avec les appareils de mesure sont exclusivement stockés sur la plateforme distante, et **en ce que** l'unité de traitement est configurée pour stocker dans la passerelle une commande spécifique à chaque instrument de mesure et définie par la plateforme distante, cette commande permettant en cas de coupure internet de stocker d'une part des données provenant desdits instruments de mesure durant la durée de la coupure et d'autre part de les restituer vers la plateforme distante une fois que la liaison internet est rétablie.

2. Système (100) selon la revendication précédente, **caractérisé en ce que** les premiers moyens de connexion (161-163) sont du type Ethernet et/ou RS232 et/ou USB et/ou sans fils, et/ou les seconds moyens de connexion (151-152) sont du type filaire et/ou sans fils.

3. Système (100) selon l'une quelconque des revendications précédentes, **caractérisé en ce que** les données envoyées vers le serveur distant (130) sont encapsulées et/ou cryptées.

4. Système (100) selon l'une quelconque des revendications précédentes, **caractérisé en ce que** la plateforme distante est configurée en outre pour directement contrôler à distance au moins un instrument de mesure.

5. Système (100) selon la revendication précédente, **caractérisé en ce que** le contrôle à distance est réalisé par un client léger du type fenêtre virtuelle émulant l'interface de contrôle dudit au moins un instrument de mesure.

6. Système (100) selon l'une quelconque des revendications précédentes, **caractérisé en ce qu'**au moins une passerelle (150) comprend un moyen de géolocalisation manuel ou automatique.

7. Système (100) selon l'une quelconque des revendications précédentes et comprenant une pluralité de passerelles (150), ledit système étant **caractérisé en ce qu'**au moins une première partie de la pluralité de passerelles (150) est agencée pour communiquer avec la plateforme distante, et **en ce qu'**au moins une deuxième partie de la pluralité de passerelles (150) est connectée à au moins une passerelle (150) de ladite première partie par les premiers moyens de connexion (161-163).

8. Procédé de transfert de données d'instruments de mesure (110-112), ledit procédé étant mis en oeuvre par le système selon l'une quelconque des revendications 3 à 7 et comprenant au moins une itération des étapes suivantes :
- Communication entre le serveur distant et les appareils de communication au moyen de protocoles de communication stockés exclusivement dans la plateforme distante,
- transfert des données d'un instrument de mesure vers la passerelle (150), par l'intermédiaire des premiers moyens de connexion (161-163),
- encapsulation desdites données de mesures par ladite passerelle (150),
- transfert des données encapsulées vers le serveur distant (130) grâce aux seconds moyens de connexion (151-152),
- en cas d'une perte de réseau internet, une commande spécifique à chaque instrument de mesure, définie par la plateforme distante et stockée préalablement sur la passerelle est utilisée, lors de son exécution, pour stocker d'une part des données provenant desdits instruments de mesure durant la durée de la coupure et d'autre part pour les restituer vers la plateforme distante une fois que la liaison internet est rétablie.

9. Procédé selon la revendication précédente, **caractérisé en ce qu'**il comprend une étape de paramétrage d'au moins un instrument de mesure par accès distant comprenant les étapes suivantes :
- affichage d'une interface graphique de type client web configurée pour définir des variables de paramétrage dudit instrument,
- transfert desdites variables de paramétrage vers la passerelle (150) par les seconds moyens de connexion (151-152) et via un protocole de transfert sécurisé,
- transfert desdites variables de paramétrage vers ledit instrument de mesure par les premiers moyens de connexion (161-163).

10. Procédé selon l'une quelconque des revendications 8 ou 9, **caractérisé en ce qu'**il comprend une étape de téléchargement sur la plateforme distante d'un protocole de mesure agencé pour communiquer avec au moins un instrument de mesure.

## Patentansprüche

1. Universelles Kommunikationssystem (100) für Messgeräte, wobei das System enthält:
- einen entfernt liegenden Server (130), enthaltend:
- Speichereinrichtungen,
- eine entfernt liegende Plattform vom Thin-Client- Typ, die dazu ausgebildet ist, mit zumindest einem Teil einer Mehrzahl von Messinstrumenten (110 - 112) zu interagieren,
- zumindest ein Gateway (150), das zwischen dem entfernt liegenden Server (130) und der Mehrzahl von Messinstrumenten (110 - 112) angeordnet ist, wobei das zumindest eine Gateway (150) enthält:
- erste Verbindungsmittel (161 - 163) zur bidirektionalen Verbindung mit der Mehrzahl von Messinstrumenten (110 - 112),
- zweite Verbindungsmittel (151 - 152) zur bidirektionalen Verbindung mit dem entfernt liegenden Server (130),
- eine Verarbeitungseinheit, die dazu eingerichtet ist, mit den Messinstrumenten (110 - 112) nach einer Mehrzahl von Kommunikationsprotokollen zu kommunizieren, wobei die Verarbeitungseinheit dazu angeordnet ist, zumindest eine logische Anwendung abzuspeichern und auszuführen,
**dadurch gekennzeichnet, dass** die Kommunikationsprotokolle zur Kommunikation mit den Messgeräten ausschließlich auf der entfernt liegenden Plattform abgespeichert sind und dass die Verarbeitungseinheit dazu eingerichtet ist, in dem Gateway einen für jedes Messinstrument spezifischen und von der entfernt liegenden Plattform definierten Befehl abzuspeichern, wobei dieser Befehl im Falle einer Internetunterbrechung gestattet, einerseits während der Dauer der Unterbrechung die von den Messinstrumenten stammenden Daten abzuspeichern und andererseits sie der entfernt liegenden Plattform weiterzuleiten, nachdem die Internetverbindung wieder hergestellt ist.

2. System (100) nach dem vorangehenden Anspruch, **dadurch gekennzeichnet, dass** die ersten Verbindungsmittel (161 - 163) vom Typ Ethernet und/oder RS232 und/oder USB und/oder drahtlos sind und/oder die zweiten Verbindungsmittel (151 - 152) vom Typ drahtgebunden und/oder drahtlos sind.

3. System (100) nach einem der vorangehenden Ansprüche, **dadurch gekennzeichnet, dass** die dem entfernt liegenden Server (130) zugesandten Daten eingekapselt und/oder verschlüsselt sind.

4. System (100) nach einem der vorangehenden Ansprüche, **dadurch gekennzeichnet, dass** die entfernt liegende Plattform ferner dazu eingerichtet ist, zumindest ein Messinstrument direkt fernzusteuern.

5. System (100) nach dem vorangehenden Anspruch, **dadurch gekennzeichnet, dass** die Fernsteuerung von einem Thin-Client mittels eines als virtuellen Fensters ausgeführt wird, welches die Steuerschnittstelle des zumindest einen Messinstruments emuliert.

6. System (100) nach einem der vorangehenden Ansprüche, **dadurch gekennzeichnet, dass** zumindest ein Gateway (150) eine manuelle oder automatische Geolokalisationseinrichtung enthält.

7. System (100) nach einem der vorangehenden Ansprüche, enthaltend eine Mehrzahl von Gateways (150), wobei das System **dadurch gekennzeichnet ist, dass** zumindest ein erster Teil aus der Mehrzahl von Gateways (150) dazu angeordnet ist, mit der entfernt liegenden Plattform zu kommunizieren, und dass zumindest ein zweiter Teil aus der Mehrzahl von Gateways (150) mit zumindest einem Gateway (150) der ersten Teils über die ersten Verbindungseinrichtungen (161 - 163) verbunden ist.

8. Verfahren zum Übertragen von Daten von Messinstrumenten (110 - 112), wobei das Verfahren von dem System nach einem der Ansprüche 3 bis 7 durchgeführt wird und zumindest eine Iteration der nachfolgenden Schritte umfasst:
- Kommunikation zwischen dem entfernt liegenden Server und den Kommunikationsgeräten mittels Kommunikationsprotokollen, die ausschließlich in der entfernt liegenden Plattform abgespeichert sind,
- Übertragen der Daten von einem Messinstrument zum Gateway (150) über die ersten Verbindungseinrichtungen (161- 163),
- Einkapseln der Messdaten mittels des Gateways (150),
- Übertragen der eingekapselten Daten an den entfernt liegenden Server (130) über die zweiten Verbindungseinrichtungen (151 - 152),
- bei Verlust des Internet-Netzwerks Verwenden eines jedem Messinstrument spezifischen Befehls, der von der entfernt liegenden Plattform definiert und zuvor auf dem Gateway abgespeichert wurde, bei seiner Ausführung, um einerseits die von den Messinstrumenten stammenden Daten während der Dauer der Unterbrechung zu speichern und andererseits sie zur entfernt liegenden Plattform weiterzuleiten, nachdem die Internet-Verbindung wieder hergestellt ist.

9. Verfahren nach dem vorangehenden Anspruch, **dadurch gekennzeichnet, dass** es einen Schritt der Parametrierung zumindest eines Messinstruments durch Fernzugang umfasst, oder die nachfolgenden Schritte aufweist:
- Anzeigen einer graphischen Benutzeroberfläche vom Typ Web-Client, die dazu ausgebildet ist, Parametrierungsvariable des Instruments zu definieren,
- Übertragen der Parametrierungsvariablen an das Gateway (150) über die zweiten Verbindungseinrichtungen (151 - 152) und über ein gesichertes Übertragungsprotokoll,
- Übertragen der Parametrierungsvariablen an das Messinstrument über die ersten Verbindungseinrichtungen (161 - 163).

10. Verfahren nach einem der vorangehenden Ansprüche 8 oder 9, **dadurch gekennzeichnet, dass** es einen Schritt des Downloadens eines Messprotokolls auf die entfernt liegende Plattform umfasst, das dazu ausgebildet ist, mit zumindest einem Messinstrument zu kommunizieren.

## Claims

1. Universal communication system (100) for measurement devices, said system comprising:
- a remote server (130) comprising:
- storage means,
- a remote platform of the thin client type, configured in order to interact with at least one part of a plurality of measurement instruments (110-112),
- at least one gateway (150) arranged between the remote server (130) and the plurality of measurement instruments (110-112), said at least one gateway (150) comprising:
- first means for bidirectional connection (161-163) with the plurality of measurement instruments (110-112),
- second means for bidirectional connection (151-152) with the remote server (130),
- a processing unit configured in order to communicate with said measurement instruments (110-112) according to a plurality of communication protocols, said processing unit being arranged in order to store and execute at least one software application,
**characterized in that** the protocols for communication with the measurement devices are exclusively stored on the remote platform, and **in that** the processing unit is configured in order to store in the gateway a command specific to each measurement instrument and defined by the remote platform, this command making it possible in the case of an internet outage on the one hand to store data originating from said measurement instruments for the duration of the outage and on the other hand to restore the data to the remote platform once the internet connection is reestablished.

2. System (100) according to the preceding claim, **characterized in that** the first connection means (161-163) are of the Ethernet and/or RS232 and/or USB and/or wireless type, and/or the second connection means (151-152) are of the wired and/or wireless type.

3. System (100) according to any one of the preceding claims, **characterized in that** the data sent to the remote server (130) are encapsulated and/or encrypted.

4. System (100) according to any one of the preceding claims, **characterized in that** the remote platform is moreover configured in order to directly remotely control distance at least one measurement instrument.

5. System (100) according to the preceding claim, **characterized in that** the remote control is carried out by a thin client of the virtual window type emulating the control interface of said at least one measurement instrument.

6. System (100) according to any one of the preceding claims, **characterized in that** at least one gateway (150) comprises a manual or automatic geolocation means.

7. System (100) according to any one of the preceding claims and comprising a plurality of gateways (150), said system being **characterized in that** at least one first part of the plurality of gateways (150) is arranged in order to communicate with the remote platform, and **in that** at least one second part of the plurality of gateways (150) is connected to at least one gateway (150) of said first part by the first connection means (161-163).

8. Method for the transfer of data from measurement instruments (110-112), said method being implemented by the system according to any one of claims 3 to 7 and comprising at least one iteration of the following steps:
- communication between the remote server and the communication devices by communication means protocols stored exclusively in the remote platform,
- transfer of data from a measurement instrument to the gateway (150), via the first connection means (161-163),
- encapsulation of said measurement data by said gateway (150),
- transfer of the encapsulated data to the remote server (130) using the second connection means (151-152),
- in case of an internet outage, a command specific to each measurement instrument, defined by the remote platform and stored beforehand on the gateway is used, when executed, on the one hand to store data originating from said measurement instruments for the duration of the outage and on the other hand to restore the data to the remote platform once the internet connection is re-established.

9. Method according to the preceding claim, **characterized in that** it comprises a step of parametering at least one measurement instrument by remote access comprising the following steps:
- displaying a graphical interface of web client type configured in order to define the parametering variables of said instrument,
- transfer of said parametering variables to the gateway (150) by the second connection means (151-152) and via a secure transfer protocol,
- transfer of said parametering variables to said measurement instrument by the first connection means (161-163).

10. Method according to any one of claims 8 or 9, **characterized in that** it comprises a step of downloading a measurement protocol, arranged in order to communicate with at least one measurement instrument, onto the remote platform.
